# EUROPEAN PATENT SPECIFICATION

(11) **EP 2 529 217 B1**
(45) Date of publication and mention of the grant of the patent: **23.10.2013**
(21) Application number: 10705091.6
(22) Date of filing: 29.01.2010
(51) Int. Cl.: G01N 33/04

(54) **ON-LINE SYSTEM, METHOD OF ITS CALIBRATION AND SIMULTANEOUS DETECTION OF ANTIBIOTIC RESIDUES AND THEIR CONCENTRATION IN MILK**
ONLINE-SYSTEM, VERFAHREN ZU DESSEN KALIBRIERUNG, SOWIE SIMULTANE DETEKTION VON ANTIBIOTIKARESTEN UND DEREN KONZENTRATION IN MILCH
SYSTÈME INTERACTIF, MÉTHODE DE SON CALIBRAGE ET DÉTECTION SIMULTANE DES RÉSIDUS ANTIBIOTIQUES ET LEUR CONCENTRATION EN LAIT

(43) Date of publication of application: 05.12.2012
(73) Proprietor: Tartu Ülikool (University Of Tartu), 50090 Tartu (EE)
(72) Inventor: RINKEN, Toonika, 51011 Tartu (EE); JAANISOO, Raivo, 50415 Tartu (EE)
(74) Representative: Kahu, Sirje
(86) International application number: PCT/EE2010/000004
(87) International publication number: WO 2011/091805

(56) References cited:
- ADANYI N ET AL: "Multi-enzyme biosensors with amperometric detection for determination of lactose in milk and dairy products" 1999, EUROPEAN FOOD RESEARCH AND TECHNOLOGY, VOL. 209, NR. 3-4, PAGE(S) 220-226 , XP002582491 ISSN: 1438-2377 page 221 - page 222; figure 1
- RINKEN T ET AL: "Determination of antibiotic residues and their interaction in milk with lactate biosensor" JOURNAL OF BIOCHEMICAL AND BIOPHYSICAL METHODS, AMSTERDAM, NL LNKD- DOI:10.1016/J.JBBM.2005.04.009, vol. 66, no. 1-3, 31 March 2006 (2006-03-31), pages 13-21, XP024996757 ISSN: 0165-022X [retrieved on 2006-03-31]
- KNECHT B G ET AL: "AUTOMATED MICROARRAY SYSTEM FOR THE SIMULTANEOUS DETECTION OF ANTIBIOTICS IN MILK" ANALYTICAL CHEMISTRY, AMERICAN CHEMICAL SOCIETY, US LNKD- DOI:10.1021/AC035028I, vol. 76, no. 3, 1 February 2004 (2004-02-01), pages 646-654, XP001047318 ISSN: 0003-2700 cited in the application
- REBE RAZ SABINA ET AL: "Label-free and multiplex detection of antibiotic residues in milk using imaging surface plasmon resonance-based immunosensor." ANALYTICAL CHEMISTRY 15 SEP 2009 LNKD- PUBMED:19685910, vol. 81, no. 18, 15 September 2009 (2009-09-15), pages 7743-7749, XP002582492 ISSN: 1520-6882
- ADRIAN J ET AL: "Waveguide interrogated optical immunosensor (WIOS) for detection of sulfonamide antibiotics in milk" BIOSENSORS AND BIOELECTRONICS, ELSEVIER BV, NL LNKD- DOI:10.1016/J.BIOS.2009.04.036, vol. 24, no. 11, 15 July 2009 (2009-07-15) , pages 3340-3346, XP026150150 ISSN: 0956-5663 [retrieved on 2009-05-03]
- TAKEBA K ET AL: "Simultaneous determination of beta-lactam antibiotics in milk by ion-pair liquid chromatography" JOURNAL OF CHROMATOGRAPHY, ELSEVIER SCIENCE PUBLISHERS B.V, NL LNKD- DOI:10.1016/S0021-9673(97)01261-2, vol. 812, no. 1-2, 3 July 1998 (1998-07-03), pages 205-211, XP004127043 ISSN: 0021-9673
- RINKEN T ET AL: "Dynamic model of amperometric biosensors. Characterisation of glucose biosensor output." January 2001 (2001-01), BIOSENSORS & BIOELECTRONICS JAN 2001 LNKD- PUBMED:11261853, VOL. 16, NR. 1-2, PAGE(S) 53 - 59 , XP002582493 ISSN: 0956-5663 cited in the application page 55 - page 58

## Description

### TECHNICAL FIELD

The present invention belongs to the field of assessment of milk quality and determination of residues, present in milk.

### BACKGROUND ART

Antibiotics are used for the treatment of inflammatory processes and in some countries as feed additives in agricultural production. The misuse of antibiotics can lead to their occurrence in milk and accordingly the contamination of milk, which in turn can cause health problems and risks of developing microbial resistance; - but also problems in dairy industry by influencing the processes of fermentation. To avoid problems, connected with milk contamination, there have been established maximum residue levels (MRL) for antibiotics in milk (European Council Regulation No. 2377/90).

For the determination of antibiotic residues in milk the following prior art methods are applied:
a) microbial inhibition tests (Delvotest, BrTest etc.; EP0005891, US5454805, EP0285792, EP1216307) for screening and qualitative analysis in farms. These tests are based on colorimetric reactions, taking place during growth of microorganisms, used in the tests (testing time 2 - 3 hours);
b) "*rapid tests*", allowing the detection of mainly β-lactam antibiotics within 10 - 15 minutes, based on different technologies (Penzym, Betastar, Charm II tests; EP0538447, US6043048);
c) chromatographic analyses, enabling both qualitative and quantitative determination of antibiotic residues.

These analyses are very expensive and time-consuming (the pre-treatment of assays and their analysis takes 1 - 2 days).

Antibiotic residues can be also determined with QCM (Park et al., Biosensors and Bioelectronics. 19, 667-674, 2004) or SPR (Gaudin et al., Analytica Chimica Acta. 436, 191-198, 2001) biosensor based technologies.

None of the above-mentioned methods or tests allow the detection of antibiotic residues or the determination of their concentrations in real time, i.e. during the time of milking the cow and do not enable *on-line* separation of substandard milk.

The most adjacent to the present invention is the antibiotics detection system, based on the application of haptens as a bio-recognition system in biosensor array (Knecht et al., Analytical Chemistry. 76, 646-654, 2004). In this biosensor array, different monoclonal antibodies, are immobilized on a glass substrate. These antibodies, selective towards particular antibiotics, generate chemiluminescence signals in the presence of antibiotics. This array enables simultaneous detection of 10 different antibiotics. The system is highly sensitive and it takes about 5 minutes for the detection of antibiotics. The major disadvantage of the system is the low speed of system regeneration, so it is not applicable for *on-line* arrangements.

### DISCLOSURE OF THE INVENTION

The present invention proposes an *on-line* system, method of its calibration and simultaneous detection of antibiotic residues and their concentrations in milk. The invention enables evaluation of milk quality in real time and on-line separation of substandard milk to avoid pollution of dairy production.

The first object of the invention is an on-line system for simultaneous detection of antibiotics and determination of their concentrations in milk, which comprises milk inflow and outflow channels and thermostated parallel identical milk flow channels, each of which is containing an oxygen sensor integrated with a different enzyme and altogether forming a biosensor array. The system comprises a signal processing device, which is connected to means for separation of substandard milk. To enable the simultaneous detection of different antibiotics and the determination of their concentrations *on-line* within the milking time, the system comprises an additional parallel milk flow channel including an enzyme-free oxygen sensor. All oxygen sensors, used in the system disclosed herein, can be optical, amperometric or potentiometric. The system comprises also the thermostated hydrolysis device located between milk inflow channel and parallel milk flow channels, which includs physical and/or chemical means to stimulate lactose hydrolysis for the generation of equal predetermined glucose and galactose concentrations in all milk samples. The physical means can be ultrasound, impulse electric field or magnetic field instrument; the chemical means can be a catalyst (like β-galactosidase or other enzyme catalysing the hydrolysis of lactose) or a compound, increasing ionic strength of the solution. There is a different enzyme catalysing the oxidation of glucose and galactose (glucose oxidase, galactose oxidase or some other enzyme belonging to the class of oxidoreductases) attached to every oxygen sensor in the biosensor array, catalytic activity thereof increases or decreases when antibiotics are present in the milk sample. The amount of the enzyme must be sufficient to generate the transient phase decrease of oxygen concentration, which is measured with given (predetermined) precision before the analysed milk is mixed with high quality milk.

The second object of the invention is the method for the calibration of the above disclosed system, comprising the following steps:
a) lactose in antibiotics-free milk sample and in milk sample, which is contaminated with definite amounts of different antibiotics, is quickly hydrolysed to generate given (predetermined) equal concentrations of lactose hydrolysis products glucose and galactose, exceeding the concentrations of glucose and galactose in raw milk for at least 10 times. This hydrolysis enables to obtain equal glucose and galactose concentrations in all milk samples, as the typical lactose concentration in milk is very stable (140±3 mM) and exceeds the typical concentrations of glucose and galactosein milk more than 1000 times;
b) each milk sample obtained by step a) (i.e. samples, in which the glucose and galactose concentrations have achieved the given (predetermined) values after lactose hydrolysis), is directed into parallel milk flow channels, where due to the catalytic activity of enzymes, present in the biosensors, glucose and galactose are oxidised by the molecular dissolved oxygen in milk and the transient phase decreases of oxygen concentration during the oxidation reactions in every parallel milk flow channel are measured with the biosensor array in each milk sample;
c) the initial concentration of molecular dissolved oxygen in each milk sample obtained by step a) is measured simultaneously with enzyme-free oxygen sensor;
d) for antibiotics-free milk samples a steady state parameter and a kinetic parameter are calculated for each reaction, going on in a different biosensor and catalyzed by the enzyme, present in the same biosensor, on the basis of the transient phase decrease of oxygen concentration during the oxidation reactions of glucose and galactose and the concentration of molecular dissolved oxygen, measured with enzyme-free oxygen sensor, and these calculated parameters for different biosensors are combined to form a characteristic pattern for antibiotics-free milk sample;
e) for milk samples, contaminated with definite amounts of different antibiotics, a steady state parameter and a kinetic parameter are calculated for each reaction, going on in a different biosensor and catalyzed by the enzyme, present in the same biosensor on the basis of the transient phase decrease of oxygen concentration during the oxidation reactions of glucose and galactose and the concentration of molecular dissolved oxygen, measured with enzyme-free oxygen sensor. Based on the combination of these calculated parameters for different biosensors a characteristic pattern or a fingerprint for every antibiotic and its concentration is formed.

The next object of the invention disclosed herein is an *on-line* method for the simultaneous detection of different antibiotics and determination of their concentrations in milk using with the above disclosed system.

The method comprises the following steps:
a) lactose in thermostated milk samples under investigation is quickly hydrolysed to obtain given (predetermined) equal concentrations of glucose and galactose, exceeding the concentrations of glucose and galactose in raw milk for at least 10 times;
b) transient phase decreases of oxygen concentration during the oxidation reactions of glucose and galactose are measured with the biosensor array in the milk sample under investigation;
c) the concentration of molecular dissolved oxygen in the milk sample under investigation is simultaneously measured with enzyme-free oxygen sensor;
d) for milk samples under investigation, a steady state parameter and a kinetic parameter are calculated for every reaction, going on in a different biosensor and catalyzed by the enzyme, present in the same biosensor on the basis of the transient phase decrease of oxygen concentration during the oxidation reactions of glucose and galactose and the concentration of molecular dissolved oxygen, measured with enzyme-free oxygen sensor, and based on the the combination of all these parameters for different biosensors a characteristic pattern for the milk sample under investigation is formed;
e) the pattern of a milk sample under investigation is compared with the calibrated fingerprints of different antibiotics and in case the pattern of the sample under investigation and the calibrated antibiotic fingerprint are identical or similar, the presence of antibiotic residue(s) isdetected;
f) the concentration of the antibiotic residue in the milk sample under investigation is calculated from the range of the change of parameters, forming the fingerprint of the antibiotic;
g) in case the concentration of an antibiotic exceeds its given concentration, the signal processing device activates the means for *on-line* separation of substandard milk. The given (predetermined) concentration of antibiotics in milk samples under investigation is determined according tolaw.

Disclosed in the present invention *on-line* system, method for its calibration and simultaneous detection of different antibiotics and determination their concentrations in milk have several advantages in comparison with prior art solutions, as they allow
- to detect potential antibiotics' residues in milk *on-line;*
- to determine the quality of milk in real time;
- to remove substandard milk before milk collecting tank. Application of the system is simple, doesn't require special training, can be fully automated, enables data saving and the system can be used repeatedly. If required, the system can be easily regenerated and renewed in a couple of minutes.

### FIGURES

Fig. *On-line* system for the simultaneous detection of different antibiotics and determination their concentrations in milk
1 - milk inflow channel
2 - parallel identical milk flow channels
3 - oxygen sensor with attached enzyme
4 - biosensor array
5 - means for separation of substandard milk
6 - signal processing device
7 - milk outflow channel
8 - enzyme-free oxygen sensor
9 - device of hydrolysis

### DESCRIPTION OF EMBODIMENTS

For the detection of antibiotics in milk we constructed a system, calibrated it towards different studied antibiotics and measured the concentrations of different antibiotics in milk samples.

The system comprised milk inflow channel 1 and thermostated parallel milk flow channels 2, every one of which is containing an oxygen sensor 3, integrated with a different enzyme and together forming a biosensor array 4. For the integration of enzymes, catalysing the oxidation of glucose and galactose, we immobilised different enzymes onto insoluble carrier and attached them with this carrier to every oxygen sensor 3 in biosensor array 4. In a parallel channel to the above-described parallel identical milk flow channels 2, there was an enzyme-free oxygen sensor, to which we attached enzyme-free carrier, similar to those used for enzyme immobilization.

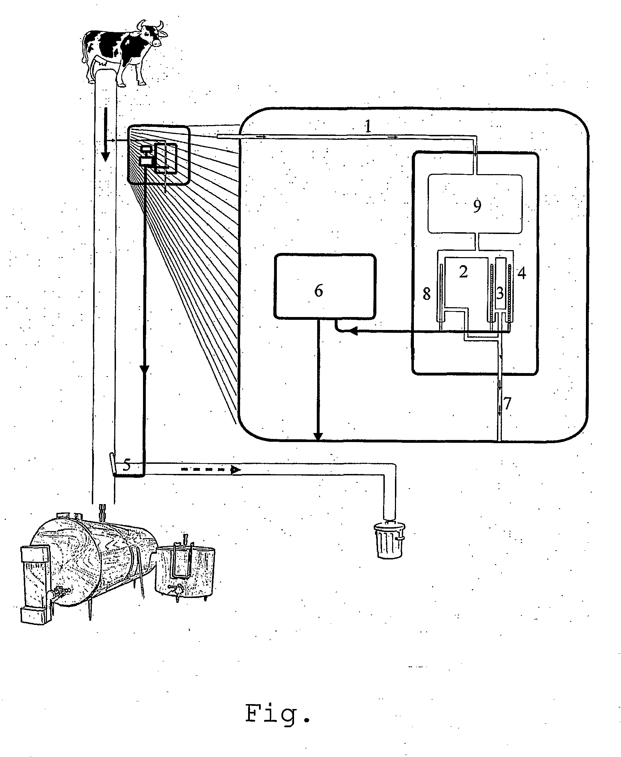

The system comprised also means for separation of substandard milk 5, connected to a signal processing device 6, milk outflow channel 7 and a device of hydrolysis 9 located between milk inflow channel 1 and parallel milk flow channels 2 (Figure 1).

Enzymes, used in biosensors, were glucose oxidase (GOD, EC 1.1.3.4.) and galactose oxidase (GAO, EC 1.1.3.9). These enzymes were covalently immobilised on nylon-6,6 threads with the help of dimethylsulfate and glutaric aldehyde. Nylon threads containing immobilized enzymes were coiled around the outer surface of an optical oxygen sensor (EE04250 B1). We used 25 cm long threads of the abovementioned enzymes, which specific activities were (0.014 ± 0.001) IU/cm and (0.63 ± 0.02) IU/cm, accordingly. 25 centimetres of enzyme-free carrier was coiled around the outer surface of the enzyme-free oxygen sensor 8.

Fiberoptical oxygen sensors were used as oxygen sensors 3 and enzyme-free sensors 8.

Located between milk inflow channel 1 and parallel milk flow channels 2 was the device of hydrolysis 9, where ultrasound was applied to increase the speed of lactose hydrolysis catalysed by β-galactosidase (β-GAL, EC 3.2.1.23).

The system was thermostated at 38°C.

### Calibration of the system

### a) lactose hydrolysis

For the accelerated hydrolysis of milk lactose into glucose and galactose, we used ultrasound to increase the speed of enzymatic hydrolysis, catalysed by β-galactosidase, which concentration was 10.1 IU/ml. For partial lactose hydrolysis we used ultrasound for 30 seconds (average output 12.6 W). This resulted in the hydrolysis of 1% of lactose and the concentrations of glucose and galactose in milk samples increased to 1.4 mM. Applying higher or lower ultrasound outputs, the acceleration of lactose hydrolysis was smaller. Ultrasound also caused the decrease of dissolved oxygen concentration in milk samples, but the remaining amount of the dissolved oxygen in samples was enough for the oxidation of glucose and galactose. In some cases we also added 1.6 M NaCl for the acceleration of lactose hydrolysis.

Acceleration of lactose enzymatic hydrolysis by impulse electric field or magnetic field was less effective than by ultrasound.

We studied the speed of lactose hydrolysis, the kinetics of glucose and galactose oxidation reactions in biosensor array 4 and the sensitivity of biosensors 3 at lower temperatures (4 - 38°C) than 38°C, used in the system. Although the analysis time was substantially longer at lower temperatures than on the higher ones, it was possible to use the system even at 4°C (milk temperature in milk tanks), as milk is kept in milk tanks for longer periods.

### b) measuring oxygen concentration

Hydrolysed milk samples were directed to parallel milk flow channels 2: in the first channel there was a biosensor comprising fiberoptical oxygen sensor 3 and glucose oxidase; in the second channel there was a biosensor comprising fiberoptical oxygen sensor 3 and galactose oxidase; and in the third channel there was an enzyme-free fiberoptical oxygen sensor 8.

When the parallel milk flow channels 2 were filled with milk samples, the milk outflow channel 7 was closed and measuring of the oxygen concentration in the sample started. In milk flow channels 2, which were containing biosensors, the concentration of dissolved oxygen began to decrease due to the oxidation reactions catalysed by the immobilised enzymes in biosensors, forming biosensor array 4. As the enzymes had different selectivity towards glucose, galactose and lactose, the decrease of oxygen concentration was different in different milk flow channels.

The concentration of dissolved oxygen in milk samples, entering the milk flow channels, was measured with an enzyme-free oxygen sensor 8. The decrease of oxygen in the milk flow channels was measured for 20 seconds. Within this time period, the concentration of dissolved oxygen in antibiotics-free milk samples decreased at least 5%.

c) calculating reaction steady state and kinetic parameters and combinating these reaction parameters to form the characteristic pattern for antibiotics-free milk samples and to form patterns or fingerprints for milk samples, which are contaminated with definite amounts of different antibiotics

A steady state parameter (parameter *A*) and a kinetic parameter (parameter *B*) were calculated for every reaction, going on in a different biosensor for every milk sample from the transient phase decrease of oxygen concentration caused by the oxidation reactions and the concentration of molecular dissolved oxygen, measured with enzyme-free oxygen sensor. We used the dynamic model of biosensors (Rinken and Tenno, *Biosensors* & *Bioelectronics.* **16**, 53-59, 2001), which was complemented with factors characterizing fiberoptical oxygen sensors, for the calculation of the abovementioned parameters, as this model enables the calculation of reaction steady state and kinetic parameters from the biosensor transient phase signal data.

Combining parameters *A* and *B* for different biosensors for milk samples, where the concentrations of glucose and galactose were 1.4 mM, we got the pattern for antibiotics-free milk samples (in our system GOD biosensor A = 0.159 and B = 222.2 sec⁻¹; GAO biosensor A = 0.640 and B = 53.0 sec⁻¹).

When a definite amount of antibiotic (benzylpenicillin, streptomycin or chloramphenicol) was added to a milk sample, the combinations of reaction parameters changed - some parameters increased and some decreased depending on the nature of the added antibiotic and the essence of the parameter (parameter *A* or parameter *B*). For example, the values of GOD biosensor parameters *A* and *B* both decreased with the increase of benzylpenicillin concentration in milk samples (for example at benzylpenicillin concentration 1000 ppb the value of steady state parameter *A* was 0.141 and the value of kinetic parameter *B* was 146.0 sec⁻¹); but as for GAO biosensor, the value of parameter *A* decreased and the value of parameter *B* increased (for example at benzylpenicillin concentration 1000 ppb the value of steady state parameter *A* was 0.435 and the value of kinetic parameter *B* was 97.8 sec⁻¹).

The range of change the values of these parameters for different biosensors were studied also in case, when streptomycin, chloramphenicol or benzylpenicillin and streptomycin mixtures were added to milk samples.

### Detection of antibiotic residues and determination of their concentrations in milk

The system was applied for the analysis the milk samples of cows, treated with antibiotics (Penstrep muscle injections) and whose milk was tested positive to antibiotics with Delvotest. The last treatment with antibiotics was carried out 24 - 72 hours earlier.

The results of milk analysis with the above-described system and Delvotest were in good correlation. The average time for the analysis with the system was 30 - 40 seconds, allowing on-line separation of substandard milk before its mixing with high quality milk.

The concentration of antibiotic residues in milk, which is considered substandard, is determined according to the number of animals, whose milk is collected to the same tank, but also considering the economic calculations. An optimum concentration of a certain antibiotic in the milk of one cow is about 10 times higher than the MRL for the same antibiotic.

The embodiments described herein illustrate the principles of the invention and are not intended to be exhaustive.

## Claims

1. System for detection of antibiotic residues and determination of their concentrations in milk comprising milk inflow channel 1 and thermostated parallel milk flow channels 2, each of which is containing an oxygen sensor 3, integrated with a different enzyme and altogether forming a biosensor array 4, and means for separation of substandard milk 5, connected to a signal processing device 6, and milk outflow channel 7,
**characterized in that** for simultaneous detection of different antibiotics and determination of their concentrations on-line within milking time, the system comprises an additional parallel milk flow channel 2 including an enzyme-free oxygen sensor 8 and hydrolysis device 9 located between milk inflow channel 1 and parallel milk flow channels 2, and said hydrolysis device 9 comprises physical and/or chemical means for the controlled hydrolysis of lactose to obtain predetermined equal glucose and galactose concentrations in every milk sample under investigation, and each biosensor 3 in biosensor array 4 is integrated with a different enzyme, which catalyzes the oxidation of glucose and galactose by molecular dissolved oxygen, and the catalytic activity of said enzymes increases or decreases in the presence of antibiotics, and the amount of said enzymes is sufficient to generate the transient phase decrease of oxygen concentration, and signal processing device 6 is adapted for activation of means for on-line separation of substandard milk before the analysed milk is mixed with high quality milk, and wherein the system is adapted for simultaneous calculation of the transient phase decrease of oxygen concentration in milk during the oxidation of glucose and galactose in different milk flow channels 2 and for comparison of the pattern of a milk sample under investigation with the calibrated fingerprints of different antibiotics.

2. System of claim 1, **characterized in that** the physical means used in the hydrolysis device 9 is an ultrasound, impulse electric field or magnetic field instrument.

3. System of claim 1, **characterized in that** the chemical means used in the hydrolysis device 9 is a catalyst or a compound, increasing ionic strength of a solution.

4. System of claim 3, **characterized in that** the named catalyst is β-galactosidase or any other enzyme, catalyzing the hydrolysis of lactose.

5. System of claim 1, **characterized in that** each oxygen sensor 3 in biosensor array 4 is integrated with a different enzyme, which is glucose oxidase, galactose oxidase or any other oxidoreductase.

6. System of claim 1, **characterized in that** oxygen sensor 3 in biosensor array 4 and enzyme-free oxygen sensor 8 is optical, amperometric or potentiometric.

7. Method for the calibration of the system of claim 1 comprising the following steps:
a) hydrolysing lactose in antibiotics-free milk sample and in milk sample contaminated with definite amounts of different antibiotics for obtaining predetermined equal concentrations of glucose and galactose, exceeding the concentrations of glucose and galactose in raw milk at least 10 times;
b) measuring the transient phase decreases of oxygen concentration during the oxidation reactions of glucose and galactose with the biosensor array in antibiotics-free milk sample and in every milk sample contaminated with definite amounts of different antibiotics;
c) measuring simultaneously the concentration of molecular dissolved oxygen in every milk sample with enzyme-free oxygen sensor;
d) based on the measurement results of steps b) and c) calculating for antibiotics-free milk sample a steady state parameter and a kinetic parameter of enzymatically catalyzed reactions of each biosensor, and combining of all said parameters to form a characteristic pattern for antibiotics-free milk sample;
e) based on the measurement results of steps b) and c) calculating for milk samples, contaminated with definite amounts of different antibiotics, a steady state parameter and a kinetic parameter of enzymatically catalyzed reaction of each biosensor and combining of all these parameters to form a characteristic pattern or a fingerprint for each antibiotic.

8. On-line method for the simultaneous detection of different antibiotics and determination of their concentrations in milk with the system of claim 1, comprising milking the animal and taking a milk sample, which is thermostated and **charact erized** in that the method is comprising the following steps:
a) quickly hydrolyzing lactose in milk sample analysed to obtain predetermined equal concentrations of glucose and galactose, exceeding the concentrations of glucose and galactose in raw milk at least 10 times;
b) measuring the transient phase decreases of oxygen concentration during the oxidation reactions of glucose and galactose the biosensor array in the milk sample under investigation;
c) measuring simultaneously the concentration of molecular dissolved oxygen in the milk sample under investigation with enzyme-free oxygen sensor;
d) based on the measurement results of steps b) and c) calculating for milk sample under investigation a steady state parameter and a kinetic parameter for enzymatically catalyzed reaction of each biosensor and combining of all these parameters to form a characteristic pattern for the milk sample analysed;
e) comparing the pattern of a milk sample under investigation with the calibrated fingerprints of different antibiotics and detecting the presence of antibiotic residue(s) in case the pattern of the milk sample under investigation and the calibrated antibiotic fingerprint are identical or similar;
f) calculating the concentration of the antibiotic residue in the milk sample from the range of the change of parameters, forming the fingerprint of the antibiotic;
g) activating the means of on-line separation of substandard milk by the device for signal processing if the concentration of an antibiotic(s) in the milk sample under investigation exceeds its given concentration.

## Patentansprüche

1. Online-System zur Detektion von Antibiotikaresten und deren Konzentration in Milch, umfasst einen Milcheinlaufkanal (1) und parallele Milchflusskanäle (2) mit Temperaturregelung, jeder von diesen mit einem Sauerstoffsensor (3), mit einem unterschiedlichen Enzym versehen und zusammen ein Biosensorreihe bildend (4), sowie Mitteln zur Trennung nicht normgerechter Milch (5), verbunden mit einem Signalverarbeitenden Gerät (6), und einem Milchausflusskanal (7), **dadurch gekennzeichnet, dass** zur gleichzeitigen Detektion verschiedener Antibiotika und deren Konzentration online während des Melkens das System einen weiteren zusätzlichen Milchflusskanal (2) umfasst, zudem einen enzymfreien Sauerstoffsensor (8) und eine Hydrolysevorrichtung (9) zwischen dem Milcheinflusskanal (1) und den parallelen Milchflusskanälen (2), die genannte Hydrolysevorrichtung (9) beinhaltet physikalische und/oder chemische Mittel zur kontrollierten Hydrolyse von Laktose, um vorbestimmte gleiche Glukose- und Galaktosekonzentrationen in jeder zu untersuchenden Milchprobe zu erhalten, jeder Sauerstoffsensor (3) im Biosensorreihe (4) ist mit einem unterschiedlichen Enzym versehen, welches die Oxidation von Glukose und Galaktose über im Milch molekular gelösten Sauerstoff katalysiert, wobei die katalysierende Wirkung der genannten Enzyme bei Vorhandensein von Antibiotika zu- oder abnimmt, und die Menge der genannten Enzyme ausreicht, um eine vorübergehende Abnahme der Sauerstoffkonzentration zu erzeugen, das Signalverarbeitungsgerät (6) ist darauf abgestimmt, Mittel zur on-line-Trennung von minderwertiger Milch zu aktivieren, bevor die analysierte Milch mit qualitativ hochwertiger Milch vermischt wird, wobei das System auf die gleichzeitige Berechnung der vorübergehenden Abnahme der Sauerstoffkonzentration in der Milch während der Oxidation von Glukose und Galaktose in den unterschiedlichen Milchflusskanälen (2) abgestimmt ist, ebenso auf einen Vergleich der Musters der zu untersuchenden Milchprobe mit den kalibrierten Fingerabdrücken verschiedener Antibiotika.

2. Online-System nach Anspruch 1, welches sich **dadurch gekennzeichnet, dass** das physikalische Mittel, welches in der Hydrolysevorrichtung (9) verwendet wird, ein Ultraschall-, Impulselektrofeld- oder Magnetfeldinstrument ist.

3. Online-System von Anspruch 1, welches sich **dadurch gekennzeichnet, dass** das chemische Mittel, welches in der Hydrolysevorrichtung (9) verwendet wird, ein Katalysator oder eine Verbindung ist, welche die Ionenstärke der Lösung vergrößert.

4. Online-System nach Anspruch 3, welches sich **dadurch gekennzeichnet, dass** es sich bei dem genannten Katalysator um B-Galaktosidase handelt, oder irgendein anderes Enzym, welches die Hydrolyse von Laktose katalysiert.

5. Online-System nach Anspruch 1, welches sich **dadurch gekennzeichnet, dass** jeder Sauerstoffsensor (3) in der Biosensorreihe (4) mit einem unterschiedlichen Enzym versehen ist, wobei es sich um Glukoseoxidase, Galaktoseoxidase oder eine andere Oxioreduktase handelt.

6. Online-System nach Anspruch 1, welches sich **dadurch gekennzeichnet, dass** der Sauerstoffsensor (3) in der Biosensorreihe (4) und enzymfreie Sauerstoffsensor (8) optisch, amperometrisch oder potentiometrisch ist.

7. Verfahren zur Kalibrierung des Online-Systems nach Anspruch 1 wobei das Verfahren die folgenden Schritte umfasst:
a) hydrolysieren von Laktose in antibiotikafreier Milch und in einer Milchprobe, die mit bestimmten Mengen verschiedener Antibiotika kontaminiert ist, um vorbestimmte gleiche Konzentrationen von Glukose und Laktose zu erhalten, die die Konzentrationen von Glukose und Laktose in Rohmilch um mindestens das Zehnfache übersteigen;
b) Messung der vorübergehenden Abnahme der Sauerstoffkonzentration während der Oxidationsreaktionen von Glukose und Galaktose vor Gleichgewichtszustand innerhalb des Biosensorreihe in einer Probe von antibiotikafreier Milch und jeder Milchprobe, welche mit bestimmten Mengen verschiedener Antibiotika kontaminiert wurde;
c) gleichzeitige Messung der Konzentration molekular gelösten Sauerstoffs in jeder Milchprobe mittels eines enzymfreien Sauersoffsensors;
d) auf Grundlage der Messergebnisse in Schritten b) und c) für die Probe antibiotikafreier Milch eine Berechnung der Parameter im Ruhezustand und im Bewegungszustand der enzymatisch katalysierten Reaktionen jedes Biosensors, sowie eine Kombination aller Parameter zum Erhalt eines charakteristischen Musters für eine antibiotikafreie Milchprobe;
e) auf Grundlage der Messergebnisse in Schritten b) und c) für Milchproben, die mit bestimmten Mengen verschiedener Antibiotika kontaminiert wurden, eine Berechnung der Parameter im Ruhezustand und im Bewegungszustand der enzymatisch katalysierten Reaktionen jedes Biosensors, sowie eine Kombination aller Parameter zum Erhalt eines charakteristischen Musters für jedes Antibiotikum.

8. Online-Verfahren zur gleichzeitigen Detektion verschiedener Antibiotika und die Bestimmung von deren Konzentration in Milch durch das System nach Anspruch 1, welche das Melken des Tieres und die Entnahme einer Milchprobe umfasst, die thermostatiert wird, und welche sich **dadurch gekennzeichnet, dass** das Verfahren die folgenden Schritte umfasst:
a) eine schnelle Hydrolyse von Laktose in der analysierten Milchprobe zum Erhalt einer vorbestimmten gleichen Konzentration von Glukose und Galaktose, die die Konzentrationen von Glukose und Laktose in Rohmilch um mindestens das Zehnfache übersteigen;
b) Messung der vorübergehenden Abnahme der Sauerstoffkonzentration während der Oxidationsreaktionen von Glukose und Galaktose vor Gleichgewichtszustand innerhalb der Biosensorreihe in der zu untersuchenden Milchprobe;
c) gleichzeitige Messung der Konzentration molekular gelösten Sauerstoffs in der zu untersuchenden Milchprobe mittels eines enzymfreien Sauerstoffsensors;
d) auf Grundlage der Messergebnisse in Schritten b) und c) für die zu untersuchende Milchprobe eine Berechnung der Parameter im Ruhezustand und im Bewegungszustand der enzymatisch katalysierten Reaktionen jedes Biosensors, sowie eine Kombination aller Parameter zum Erhalt eines charakteristischen Musters für die zu untersuchende Milchprobe;
e) ein Vergleich des Musters einer zu untersuchenden Milchprobe mit den kalibrierten Fingerabdrücken verschiedener Antibiotika und der Detektion des Vorhandenseins von Antibiotikaresten, falls das Muster der zu untersuchenden Milchprobe und der kalibrierte antibiotische Fingerandruck identisch oder ähnlich sind;
f) Berechnung der Konzentration der Antibiotikarückstände in der Milchprobe aus der Menge der Parameteränderungen, welche den Fingerabdruck des Antibiotikums bilden;
g) Aktivierung des Mittels zur Online-Abtrennung minderwertiger Milch durch das Signalverarbeitungsgerät, falls die Konzentration von Antibiotika in der zu untersuchenden Milchprobe die vorgegebene Konzentration übersteigt.

## Revendications

1. Système de détection simultanée des résidus antibiotiques et leur concentration en lait comprenant un canal d'alimentation du lait (1) et des canaux d'alimentation du lait parallèles thermorégulés (2), chacun desdits canaux d'alimentation contenant un capteur d'oxygène (3), intégré avec un enzyme différent, qui forment ensemble un réseau de biocapteurs (4), et des moyens (5) pour séparer le lait impropre, connectés au dispositif de traitement de signaux (6), ainsi qu'un canal de sortie (7), **caractérisé en ce que** pour la détection simultanée des antibiotiques différents et pour la détermination de leur concentrations intégrée avec le temps de traite, le système comprend un canal supplémentaire parallèle (2) comprenant un capteur d'oxygène (8) sans enzymes et un dispositif d'hydrolyse (9) situé entre le canal d'alimentation du lait (1) et les canaux d'alimentation du lait parallèles (2), et ledit dispositif d'hydrolyse (9) comprend des moyens physiques et/ou chimiques pour une hydrolyse de lactose contrôlée pour obtenir les concentrations prédéterminées égales du glucose et du galactose dans chaque échantillon de lait étudié, et chaque biocapteur (3) dans le réseau des biocapteurs (4) est intégré avec un enzyme différent qui catalyse l'oxydation du glucose et du galactose avec l'oxygène moléculaire dissous, et l'activité catalytique desdits enzymes augmente ou diminue en présence d'antibiotiques, et la quantité desdits enzymes est suffisante pour générer une phase de baisse transitoire de la concentration de l'oxygène, et le dispositif de traitement de signaux (6) est adapté pour l'activation des moyens de la séparation interactive du lait impropre avant que le lait analysé soit mélangé avec du lait de haute qualité, et où le système est adapté pour le calcule simultané de la phase de baisse transitoire de la concentration de l'oxygène en lait pendant l'oxydation du glucose et du galactose dans les canaux d'alimentation de lait (2) différents et pour la comparaison du parcours de l'échantillon de lait étudié avec les empreintes étalonnées des antibiotiques différents.

2. Système selon la revendication 1, **caractérisé en ce que** le moyen physique utilisé dans le dispositif d'hydrolyse (9) est une échographie, ou un instrument d'impulsion du champ électrique ou du champ magnétique.

3. Système selon la revendication 1, **caractérisé en ce que** le moyen chimique utilisé dans le dispositif d'hydrolyse (9) est un catalyseur ou un composé augmentant la force ionique d'une solution.

4. Système selon la revendication 3, **caractérisé en ce que** ledit catalyseur est un β-galactosidase ou autre enzyme catalysant l'hydrolyse de lactose.

5. Système selon la revendication 1, **caractérisé en ce que** chaque capteur d'oxygène (3) dans le réseau de biocapteurs (4) est intégré avec un enzyme différent, l'oxydase de glucose, l'oxydase de galactose ou autre oxydoréductase.

6. Système selon la revendication 1, **caractérisé en ce que** le capteur d'oxygène (3) dans le réseau de biocapteurs (4) et le capteur d'oxygène (8) sans enzyme est optique, ampérométrique ou potentiométrique.

7. Méthode pour la calibration du système selon la revendication 1 comprenant les étapes suivantes :
a) hydrolyser le lactose dans l'échantillon de lait sans antibiotiques et dans l'échantillon de lait contaminé avec des quantités définies d'antibiotiques différents pour obtenir les concentrations prédéterminées égales du glucose et du galactose dépassant par au moins 10 fois les concentrations du glucose et du galactose en lait ;
b) mesurer la phase de baisse transitoire de la concentration de l'oxygène pendant les réactions d'oxydation du glucose et du galactose avec le réseau de biocapteurs dans l'échantillon de lait sans antibiotiques et dans chaque échantillon du lait contaminé avec des quantités définies d'antibiotiques différents ;
c) mesurer simultanément la concentration de l'oxygène moléculaire dissous dans chaque échantillon de lait avec un capteur d'oxygène sans enzymes ;
d) selon les résultats de mesure des étapes b) et c), calculer pour l'échantillon de lait sans antibiotiques un paramètre à l'état d'équilibre et un paramètre cinétique des réactions de catalyse enzymatique de chaque biocapteur, et combiner tous lesdits paramètres pour former un modèle caractéristique pour l'échantillon de lait sans antibiotiques ;
e) selon les résultats de mesure des étapes b) et c), calculer pour l'échantillon de lait contaminé avec des quantités définies d'antibiotiques différents un paramètre à l'état d'équilibre et un paramètre cinétique des réactions de catalyse enzymatique de chaque biocapteur, et combiner tous lesdits paramètres pour former un modèle caractéristique ou une empreinte pour chaque antibiotique.

8. Méthode interactive pour la détection en-ligne des antibiotiques différents et la détermination de leur concentration en lait avec le système selon la revendication 1, comprenant le traite de l'animal et prise de l'échantillon de lait, qui est thérmorégulée et **caractérisé en ce que** la méthode comprend les étapes suivantes :
a) hydrolyser rapidement le lactose dans l'échantillon de lait analysé pour obtenir les concentrations prédéterminées égales du glucose et du galactose dépassant par au moins 10 fois les concentrations du glucose et du galactose en lait cru ;
b) mesurer la phase de baisse transitoire de la concentration de l'oxygène pendant les réactions d'oxydation du glucose et du galactose avec le réseau de biocapteurs dans l'échantillon de lait étudié ;
c) mesurer simultanément la concentration de l'oxygène moléculaire dissous dans l'échantillon de lait étudié avec un capteur d'oxygène sans enzymes ;
d) selon les résultats de mesure des étapes b) et c), calculer pour l'échantillon de lait étudié un paramètre à l'état d'équilibre et un paramètre cinétique pour une réaction de catalyse enzymatique de chaque biocapteur, et combiner tous lesdits paramètres pour former un modèle caractéristique pour l'échantillon de lait étudié ;
e) comparer le modèle de l'échantillon de lait étudié avec les empreintes étalonnées des antibiotiques différents et détecter la présence de résidu(s) antibiotique(s), si le modèle de l'échantillon de lait étudié et l'empreinte étalonnée de l'antibiotique sont identiques ou similaires ;
f) calculer la concentration du résidu antibiotique dans l'échantillon de lait, dans l'étendue de changements des paramètres, formant l'empreinte de l'antibiotique ;
g) activer les moyens de séparation interactive du lait de qualité inférieure par le dispositif de traitement de signaux si la concentration d'un ou plusieurs antibiotiques dans l'échantillon de lait étudié dépasse sa concentration indiquée.
